# EUROPEAN PATENT APPLICATION

(11) **EP 3 604 551 A1**
(43) Date of publication of application: **05.02.2020**
(21) Application number: 18306040.9
(22) Date of filing: 31.07.2018
(51) Int. Cl.: C12Q 1/48, A61K 39/00, G01N 33/574

(54) **COMPLEMENT-DEPENDENT CYTOTOXICITY AND SIALYLATION AS BIOMARKERS FOR PREDICTING CLINICAL RESPONSE TO CANCER TREATMENT WITH ANTIBODY OR DERIVATIVE MEDIATING COMPLEMENT ACTIVATION**

(71) Applicant: Université de Bretagne Occidentale, 29238 Brest (FR); Centre Hospitalier Regional Universitaire Brest, 29200 Brest (FR); INSERM (Institut National de la Santé et de la Recherche Médicale), 75013 Paris (FR)
(72) Inventor: PERS, Jacques-Olivier, 29200 BREST (FR); RENAUDINEAU, Yves, 29200 BREST (FR); BORDRON, Anne, 29480 LE RELECQ KERHUON (FR)
(74) Representative: Plasseraud IP

(57) **Abstract**

The present invention relates to the use of Complement-Dependent Cytotoxicity (CDC) and sialylation as biomarkers for predicting responsiveness to treatment with an antibody or derivative thereof mediating complement-activation, in particular an anti-CD20 monoclonal antibody such as Rituximab, in a cancer patient, in particular a chronic lymphocytic leukemia (CLL) patient. The invention includes the use of the biomarkers for selecting cancer patients, in particular CLL patients for treatment with an antibody or derivative thereof mediating complement-activation, in particular an anti-CD20 monoclonal antibody such as Rituximab.

SEQUENCE LISTING

<110>  Université de Bretagne Occidentale
       CHRU Brest
       INSERM

<120>  COMPLEMENT-DEPENDENT CYTOTOXICITY AND SIALYLATION AS BIOMARKERS
       FOR PREDICTING CLINICAL RESPONSE TO CANCER TREATMENT WITH
       ANTIBODY OR DERIVATIVE MEDIATING COMPLEMENT ACTIVATION

<130>  BEP180645 EP.

<160>  3

<170>  PatentIn version 3.5

<210>  1
<211>  21
<212>  DNA
<213>  artificial sequence

<220>
<223>  synthetic oligonucleotide primer

<400>  1
atatttacag aatggcacag g                                                21


<210>  2
<211>  23
<212>  DNA
<213>  artificial sequence

<220>
<223>  synthetic oligonucleotide primer

<400>  2
gaagacacat ttttactccc aac                                              23


<210>  3
<211>  27
<212>  DNA
<213>  artificial sequence

<220>
<223>  synthetic oligonucleotide primer

<400>  3
ctctgaagac acatttttac tcccaaa                                          27

## Description

### FIELD OF THE INVENTION

The present invention relates to the use of Complement-Dependent Cytotoxicity (CDC) and sialylation as biomarkers for predicting responsiveness to treatment with an antibody or derivative thereof mediating complement-activation, in particular IgG1 and IgG3 human monoclonal antibody or derivative such as Rituximab used in a cancer patient, in particular a chronic lymphocytic leukemia (CLL) patient. The invention includes the use of the biomarkers for selecting cancer patients, in particular CLL patients for treatment with an antibody or derivative thereof mediating complement-activation, in particular IgG1 or IgG3 human monoclonal antibody or derivative such as Rituximab.

The present invention also relates to a product comprising an antibody mediating complement-activation or derivative thereof and a sialic acid inhibitor, as a combined preparation for simultaneous, separate or sequential use in cancer therapy, preferably chronic lymphocytic leukemia (CLL) therapy.

### BACKGROUND OF THE INVENTION

Numerous therapeutic monoclonal antibodies (mAbs) directed against various tumor-associated antigens (for example, CD20, EGFR, CD38 and CD22), and mediating tumor cell killing through several mechanisms of action (direct and immune-mediated), involving the antigen binding (Fab) and/or Fc domains, have been developed and clinically approved. In particular, Rituximab (RTX), an immunoglobulin G1 (IgG1) kappa monoclonal antibody (mAb) directed against the B cell molecule CD20, was the first mAb to receive approval to be used in non-Hodgkin's lymphomas (NHL), and later in chronic lymphocytic leukemia (CLL) [1]. RTX mediates different mechanisms of B cell death including complement mediated mechanisms such as: complement-dependent cytotoxicity (CDC) and complement-dependent cell-mediated phagocytosis (CDCP) through the clearance of C3b(i) opsonized cells by macrophages; Fcγ receptor-mediated mechanisms such as antibody-dependent cellular cytotoxicity (ADCC) and antibody-dependent cell-mediated phagocytis (ADCP) (Lee CH et al., Nat. Immunol, 2017,18, 889-898); and direct cell death [5-11]. Efficacy of RTX varies from patient to patient and a higher rate of relapse has been observed in CLL patients compared to NHL [2]. Thus, the combination of RTX with the purine analogue, fludarabine, and the DNA alkylating agent, cyclophosphamide, represents the gold standard as first line treatment known as RFC protocol, although alternative agents such as bendamustine (RB protocol) or chlorambucil can be proposed in combination with RTX [3,4]. Other alternatives are the new oral specific inhibitors associated or not with anti-CD20 mAb such as RTX, and dose-reduced fludarabine containing regimens or lenalidomide associated with RTX. Choices were initially based on the ability to tolerate chemoimmunotherapy, but the emergence of non chemoimmunotherapy treatment options such as the new oral agent ibrutinib, a B cell receptor inhibitor, has led to propose these options in patients with risk factors (unmutated heavy light immunoglobulin chain [IGVH], chromosome 17p deletion, or mutations of TP53), and in older patients with comorbidities while RFC remains the first therapeutic choice to younger patients with IGVH mutations. RTX, in combination with chemotherapy, have become more and more effective for primary treatment in chronic lymphocytic leukemia (CLL).

However, relapses remain important with RTX as well as with many other therapeutic monoclonal antibodies that have proven clinical efficacy for cancer. At the same time, accurate diagnostic tools allowing physicians to precisely identify cancer patients who will benefit from mAb treatment are absent or limited and incomplete (unmutated IGVH, chromosome 17p deletion, or mutations of TP53 for RTX treatment in CLL patients). This results in relatively poor response rates and increased cancer morbidity and death. At a time when cancer therapy such as CLL therapy is moving towards chemo-free treatments, developing such diagnostic tools is therefore of great interest.

### SUMMARY OF THE INVENTION

Therapeutic efficacy of monoclonal antibodies (mAbs) directed against tumor-associated antigens is a challenge to study since they can mediate several mechanisms of tumor cell killing and the relative contribution of these different mechanisms of action to therapeutic efficacy in cancer patients has not been elucidated for the various therapeutic mAbs. Therapeutic mAbs can induce direct killing of tumor cells, and immune-mediated killing of tumor cells by two distinct processes, complement-mediated (CDC and CDCP), and Fcγ receptor-mediated (ADCC, ADCP) processes.

In particular, RTX therapeutic efficacy is a challenge to study since RTX mediates several mechanisms of B cell death such as direct cell death, ADCP, ADCC, CDC and CDCP. ADCC activity is linked to the efficiency of Natural Killer (NK) cells to kill tumor cells, and it is notably dependent on the Fc gamma receptor (FcγR)3 polymorphism V158F [12-14]. Although ADCC is suspected to be the main mechanism of action with regards to RTX therapeutic efficacy and clinical outcomes [15-17], the inventors suggested that activation of the complement pathway is also involved in the clinical response to anti-CD20 mAb such as RTX and ofatumumab [4].

Mechanisms controlling the RTX capacity to induce complement activation are multiple, incompletely understood and the results reported are conflicting [18-21]. For instance, the level of the target molecule CD20 appeared to be important, as well as the presence of complement regulatory molecules, which seem critical, based on the observations that blocking CD55 and/or CD59 functions are effective in increasing RTX-induced CDC [22, 23]. Also, complement C1q molecule recruitment into lipid rafts mediated by RTX suggests that expression of ganglioside M1 (GM1), a marker of lipid rafts, can determine the susceptibility to RTX treatment as reported in NHL [24].

The inventors hypothesized that another mechanism of resistance to RTX could be the sialylation of cell surface receptors such as CD22, CD45 and the B cell receptor. Sialic acid acts frequently via a α2-3 or α2-6 glycosidic linkage to galactose and N-acetylgalactosamine. Enzymes that support these sialylations belong to the sialyltransferases (ST) family, and they are unlikely to be expressed in normal B cells [25]. The presence of sialic acid is implicated in the recruitment of complement inhibitors [26], and, in turn, controls the mAb-capacity to induce complement activation [27].

Based on these hypotheses, the inventors discovered a correlation between the therapeutic responsiveness of a patient to a treatment comprising RTX and the *in vitro* capacity of RTX to induce the complement pathway when tested before treatment initiation. In this regard, they surprisingly discovered that capacity of RTX to induce CDC is influenced by the level of cell surface sialylation, preferably by the level of α2-6-linked sialic acid. Furthermore, a correlation between *in vitro* CDC, sialylation level and clinical response was observed, even though RTX treatment was administered in combination with other anticancer drugs (chemotherapy drugs).

Because the inventors observed that α2-6 sialylation is stable over time and heterogenous in tumor B cells from CLL and with a higher expression of α2-6 sialylated proteins observed in the RTX-induced CDC-sensitive group with therapeutic response, and because resistant capacity to CDC is not associated with distinct individual genetic groups or clinical outcome, a generalisation is possible to all IgG1 and IgG3 based antibodies used in the treatment of cancer and in particular CLL mediating their effect by complement-activation such as variant of RTX or other anti-CD20 mAb (Rösner et al., Br. J. Haematol., 2013, 161, 282-286,; van Maerten T and HagenBeek A, Semin. Hematol., 2010, 47, 199-210,), in particular Type I anti-CD20 antibodies such as Veltuzumab and Ofatumumab, the first fully human anti-CD20 monoclonal antibody (Korycka-Wolowiec et al., Expert Opin Drug Saf, 2015, 14, 1945-1959), anti-CCR7 antibodies (Cuesta-Mateos C et al., Cancer Immunol. Immunother., 2015, 64, 665-676), Alemtuzumab an anti-CD52 antibody (Pangalis GA et al., Med. Oncol. 2001, 18, 99-107), and anti-CD5 monoclonal antibodies (Klitgaard JL et al. , Br. J. Haematol., 2013, 163, 182-193).

These results could be also important for other types of cancers for which sialylation is known to be implicated in the aggressiveness of the disease, and for which immunotherapeutic drugs involving complement activation (CDC and/or CDCP) are used such as Cetuximab, an anti-EGFR mAb (Glassman PM and Balthasar, JP., Cancer Biol. Med., 2014, 1, 20-33,); Inotuzumab, an anti-CD22 mAb; and Daratumumab, an anti-CD38 mAb (Nijhof et al., Blood, 2016, 128, 959-970). Aberrant sialylation was found in ovarian cancer (Wichert B et al., Glycobiology, 2018, doi: 10.1093), in hepatocellular carcinoma (Han Y et al., J. Physiol. Biochem. 2018, doi: 10.1007) for which miR-9 inhibits the metastatic ability via targeting beta galactoside alpha-2,6-sialyltransferase 1, in colorectal cancer (Jia L et al., Dig. Dis. Sci., 2017, 62, 3447-3459), in renal carcinoma (Borzym-Kluczyk M et al., Acta Biochim. Pol., 2017, 64, 465-470), in prostate cancer (Pihikova D et al., Anal. Chim. Acta., 2016, 934, 72-79) and melanoma (Kolasińska E et al., Acta Biochim. Pol., 2016, 63, 533-541).

Therefore, the present invention provides a method for predicting the responsiveness of a cancer patient to a treatment with an antibody or derivative thereof mediating complement-activation, comprising:
- measuring the level of CDC induced by the antibody or its derivative, or the level of sialylation, in a tumor sample from the patient;
- wherein if said level of CDC is decreased compared to a control, then the cancer patient is likely to be non-responsive to said treatment, and if said level of CDC is unchanged or increased compared to a control then the cancer patient is likely to be responsive to said treatment; and/or
- wherein if said level of sialylation is increased compared to a control, then the cancer patient is likely to be non-responsive to said treatment, and if said level of sialylation is unchanged or decreased compared to a control then the cancer patient is likely to be responsive to said treatment.

The present invention also provides a method for the treatment of cancer in a patient in need thereof, comprising the following steps:
a. performing the method for predicting the responsiveness to treatment according to the present invention on a tumor sample from the cancer patient; and
b. if the patient is predicted as responsive to the treatment, treating said patient with the antibody or derivative thereof mediating complement-activation.

The present invention also provides a product containing an antibody or derivative thereof mediating complement-activation, preferably RTX, and a sialylation inhibitor, preferably α2-6-linked sialic acid inhibitor, as a combined preparation for simultaneous, separate or sequential use in cancer therapy, preferably CLL therapy. The sialylation inhibitor may be a sialyltransferase inhibitor, preferably α2-6 sialyltransferase inhibitor as defined above.

The present invention also provides the use of antibody-induced CDC and/or sialylation as biomarkers for predicting the responsiveness of a cancer patient to a treatment with an antibody or derivative thereof mediating complement-activation. The biomarker is preferably sialylation, more preferably α2-6-linked sialic acid.

### DETAILED DESCRIPTION OF THE INVENTION

### Definitions

As used herein, the term "cancer", refers to any type of cancer which is responsive to immunotherapy with antibodies mediating complement-activation, in particular as combined therapy with another anticancer therapy (chemotherapy or non-chemotherapy). Non-limiting examples of cancers according to the invention include leukemia, in particular chronic lymphocytic leukemia (CLL); lymphoma, in particular non-Hodgkin's lymphomas (NHL); myeloma, breast cancer, ovarian cancer, colorectal cancer, prostate cancer, renal carcinoma and melanoma.

As used herein, the term "patient" denotes a mammal, preferably a human.

In the context of the invention, the term "treating" or "treatment", as used herein, means reversing, alleviating, inhibiting the progress of, or preventing the disorder or condition to which such term applies, or reversing, alleviating, inhibiting the progress of, or preventing one or more symptoms of the disorder or condition to which such term applies.

As used herein, the expression responsiveness, sensibility or sensitivity to a treatment with an antibody mediating complement-activation (CDC and/or CDCP), in particular as combined therapy with chemotherapy and non-chemotherapy specific inhibitors, means a clinical response measurable according to established guidelines. Response can be assessed using different classification methods, such as overall survival, minimal residual response, progression free survival, radiological response, as defined by RECIST, complete response, partial response, or stable disease. The clinical response can be partial or complete. For example, the response may include a decrease in tumor load or a longer time to relapse (TTR) induced by the treatment.

As used herein, "complement activation" or "complement pathway activation", refers to complement-dependent-cytotoxicity (CDC), complement-dependent cell-mediated cytotoxicity (CDCC) and complement-dependent cell-mediated phagocytosis (CDCP). Complement-dependent-cytotoxicity (CDC and it's synonym CDCC) is a direct cell lysis process that results from insertion of the membrane attack complex into the cell. In a distinct process, the deposition of opsonins, such as C3b, that are covalently bound onto the cell surface, are in turn recognized by complement receptors (CRs) on effector cells. The CRs activated by the deposited opsonins trigger complement-dependent cell-mediated cytotoxicity (CDC) and complement-dependent cell-mediated phagocytosis (CDCP).

As used herein, "antibody mediating complement activation", refers to any antibody or derivative thereof that is capable of mediating complement activation. The antibody or derivative thereof includes antibody variant or mutant, antibody fusion protein, antibody fragment, biosimilar therapeutic antibody and others. The antibody, in particular monoclonal antibody such as therapeutic monoclonal antibody, may be human, humanized or chimeric; monospecific or multispecific (for example bispecific); a human Ig isotype, in particular IgG1 or IgG3. The antibody variant may be an isotype switch variant.

As used herein, a "tumor sample" or "sample" of a patient, refers to any biological sample comprising cancer cells of said patient. A biological sample includes a tumor biopsy or body fluid (liquid biopsy). Non-limiting examples of body fluids include blood (whole-blood), cerebral spinal fluid (CSF), amniotic fluid, urine and mucosal secretions. A tumor biopsy includes bone marrow biopsy and lymph node biopsy. Sample includes swab. Samples include also processed samples that have been treated to disrupt tissue or cell structure, thus releasing cells or intracellular components into a solution which may further contain reagents (buffers, salts, detergents, enzymes and the like) which are used to prepare, using standard methods, a biological sample for analysis. In particular, processed samples include samples that have been treated by standard methods used for the isolation of cells, nucleic acids and/or proteins from biological samples. The biological material is removed from the patient by standard methods which are well-known to a person having ordinary skill in the art. The biological sample is also named "sample".

The term "biomarker" or "marker" is widespread in the art and may broadly denote a biological molecule and/or a detectable portion thereof (e.g. a nucleic acid, a peptide or a lipid such as a glycolipid) or a biological process whose qualitative and/or quantitative evaluation in an individual is predictive or informative with respect to one or more aspects of the individual's phenotype and/or genotype, such as, for example, with respect to the status of the individual. Typically, the biomarker is predictive or informative with respect to the outcome for immunotherapeutic (including combined immunotherapeutic and chemotherapeutic or new therapeutic inhibitors) treatment of a cancer in an individual. A biomarker is expressed ("expression of the biomarker") if the biomarker is detectable with methods known in the art. Therefore expression of biomarkers encompasses not only expression at nucleic acid level (DNA and/or RNA) and protein level but also protein state including expression (presence) of other biological structures on or in the cells such as glycolipids or the activity of a protein including for example, a binding activity or enzymatic activity.

Protein biomarkers may be detected and their expression levels measured using several different techniques, many of which are antibody-based. Example of such techniques include with no limitations immunoassays (Enzyme-linked immunoassay (ELISA), radioimmunoassay, chemiluminescence- and fluorescence-immunoassay) and antibody microarray-based assays.

As used herein, the terms "a", "an", and "the" include plural referents, unless the context clearly indicates otherwise. As such, the term "a" (or "an"), "one or more" or "at least one" can be used interchangeably herein.

The above definitions are applicable to the whole application, and particularly to the following description.

### Complement-dependent cytotoxicity (CDC)

The present invention provides a method for predicting the responsiveness of a cancer patient to a treatment with an antibody mediating complement-activation, comprising measuring the level of CDC induced by the antibody on a tumor sample from the patient, wherein if said level is decreased compared to a control, then the cancer patient is likely to be non-responsive to said treatment, and if said level is unchanged or increased compared to a control, then the cancer patient is likely to be responsive to said treatment.

The tumor sample is preferably collected from the patient, prior to treatment with the antibody.

The antibody may be administered, alone or in combination with another anticancer therapy, including for example chemotherapy and/or specific inhibitors. The specific inhibitors include in particular the Bruton tyrosine kinase inhibitor Ibrutinib, the PI3-kinase delta inhibitor Idelalisib, and the BCL-2 inhibitor Venetoclax.

CDC is measured by standard assays that are well-known in the art. The CDC assay is performed on cells from a tumor sample, preferably on cells isolated from the tumor sample by standard methods that are well-known in the art (see Amos PJ, et al. Methods of Cell Purification: A Critical Juncture for Laboratory Research and Translational Science. Cells, Tissues, Organs. 2011;195(1-2):26-40). For example, the cells are B lymphocytes isolated from a blood sample by standard methods that are well-known in the art.

Following stimulation of the cells with the antibody (Ab) in the presence or absence of complement (usually human serum AB or HSAB) for a time sufficient to activate the complement pathway, complement-dependent-cytotoxicity induced by the antibody is measured by standard assay, such as apoptosis assay using fluorescent label-conjugated annexin-V (AV) and propidium iodide (PI). For B lymphocytes, in particular B lymphocytes from CLL, the stimulation step with the antibody is for at least 24h. Preferably, CDC is measured using the assay disclosed in the examples.

The level of complement-dependent-cytotoxicity induced by the antibody is determined by the percentage of CDC decreased survival, for example using the formula: 100x [(% of surviving cells (AV-PI-) without Ab in HSAB)-(% of surviving (AV-PI-) cells with Ab in HSAB)]/[% of surviving (AV-PI-) cells without Ab in HSAB].

The control is preferably a sample of normal cells or cell lines, or of cells from responsive patients, from the same tissue as the tumor, for example B lymphocytes from healthy controls or responsive patients, tested in the same conditions. Alternatively, the control may be a cut-off value, for example the mean CDC value minus 2 standard deviations of a group of controls, as explained in the examples. If the level of CDC in the patient's sample is decreased compared to the control, then the cancer patient is likely to be non-responsive to said treatment, and if said level is unchanged or increased compared to the control, then the cancer patient is likely to be responsive to said treatment.

### Sialylation level

The method for predicting the responsiveness of a cancer patient to a treatment with an antibody mediating complement-activation according to the invention, comprises measuring the level of sialylation in a tumor sample from the patient; wherein if said level is increased compared to a control, then the cancer patient is likely to be non-responsive to said treatment, if not then the cancer patient is likely to be responsive to said treatment.

The tumor sample is preferably collected from the patient, prior to treatment with the antibody.

The antibody may be administered alone or in combination with another anticancer therapy including for example chemotherapy and/or specific inhibitors. The specific inhibitors include in particular the Bruton tyrosine kinase inhibitor Ibrutinib, the PI3-kinase delta inhibitor Idelalisib, and the BCL-2 inhibitor Venetoclax.

Sialylation may be assayed by standard assays that are well-known in the art. The assay may measure expression of sialylated proteins, in particular α 2-6 sialylated proteins on tumor cell surface, for example by flow cytometry or immunohistology, using specific lectins, preferably labelled. For example, α 2-6 linked sialic acid is preferably detected using SNA lectin from *Sambucus nigra.* Flow cytometry is preferably performed by Fluorescence-activated cell sorting (FACS), a specialized type of flow cytometry that provides a method for sorting a heterogeneous mixture of biological cells into two or more containers, one cell at a time, based upon the specific light scattering and fluorescent characteristics of each cell. Immunohistology is performed on solid tumor sections prepared according to standard methods that are well-known in the art.

Alternatively, the sialylation assay may measure sialyltransferase (ST), in particular α 2-6 sialyltransferase, expression (mRNA or protein) or activity levels using standard assays that are well-known in the art. For example, sialyl transferase gene-transcripts levels may be measured by real-time polymerase chain reaction using specific primers, according to standard methods that are well-known in the art. For example, α 2-6 sialyltransferase activity may be measured by ELISA (Enzyme-linked immunosorbent assay) on tumor cell lysate as described in the examples. The ELISA is a well-known solid-phase enzyme immunoassay (EIA) used to detect the presence of a substance in a liquid sample or wet sample.

The control may be a tumor sample from a responsive patient tested in the same conditions as the tested cancer patient. Preferably, the sample from the responsive patient that is used as a control is from a tumor of the same type as the tumor of the tested patient. Alternatively, the control may be a cut-off value established as the mean of value of the assay minus 2 standard deviations of control cells, that may be obtained from healthy control cells or cell lines, for α 2-6 ST activity. If the level of sialylation in the patient's sample is increased compared to the control, then the cancer patient is likely to be non-responsive to said treatment, and if said level is unchanged or decreased compared to the control, then the cancer patient is likely to be responsive to said treatment.

In some embodiments, the cancer is selected from the group consisting of leukemia, preferably chronic lymphocytic leukemia (CLL); lymphoma, preferably non-Hodgkin's lymphomas (NHL); myeloma, breast cancer, ovarian cancer, colorectal cancer, prostate cancer, renal carcinoma and melanoma, preferably CLL.

In some embodiments, the antibody mediating complement activation is a therapeutic monoclonal antibody, preferably a human or humanized, monospecific or multispecific, IgG1 or IgG3, or a derivative thereof such as antibody variant or mutant, antibody fusion protein, antibody fragment and biosimilar therapeutic antibody.

In some preferred embodiments, the antibody mediating complement activation is selected from : anti-CD20 antibodies, preferably Type I anti-CD20 antibodies such as Rituximab (RTX), Ofatumumab and Veltuzumab; anti-CCR7 mAb; anti-CD52 mAb such as Alemtuzumab,, anti-CD5 mAb, anti-CD38 mAb such as Daratumab, anti-EGFR mAb such as Cetuximab, anti-CD22 mAb such as Inotuzumab, and variants, fragments, fusion proteins or biosimilars thereof. The anti-CD20 antibodies, preferably Type I anti-CD20 antibodies, such as RTX and Ofatumomab, are capable of redistributing the CD20 in domains resistant to detergents. They generate remarkable complement activation by recruiting C1q in these microdomains. In addition, this redistribution of CD20 facilitates their association with the B-cell receptor (BCR). Preferably, the antibody mediating complement-activation is Rituximab (RTX) or variant thereof, in particular IgG3 isotype variant.

Preferably, the present invention provides a method for predicting the responsiveness of a patient suffering from chronic lymphocytic leukemia (CLL) to a treatment with Rituximab (RTX), comprising measuring the level of CDC induced by RTX or the level of sialylation in a tumor sample comprising B lymphocytes from the patient; wherein if said level of CDC is decreased or said level of sialylation is increased compared to a control, then the cancer patient is likely to be non-responsive to said RTX treatment, if not then the cancer patient is likely to be responsive to said RTX treatment. RTX is preferably administered in combination with another anticancer therapy including for example chemotherapy and/or specific inhibitors. The specific inhibitors include in particular the Bruton tyrosine kinase inhibitor Ibrutinib, the PI3-kinase delta inhibitor Idelalisib, and the BCL-2 inhibitor Venetoclax.

In some embodiments, the sample comprising the cancer cells is a whole blood sample, a biopsy or a sample of purified cancer cells. Cancer cells may be purified by methods well-known in the art (see Amos PJ, et al. Methods of Cell Purification: A Critical Juncture for Laboratory Research and Translational Science. Cells, Tissues, Organs. 2011;195(1-2):26-40).

In some embodiments, the level of sialylation of cancer cells from the tumor sample is determined by measuring cell-surface sialylation, preferably by flow cytometry or immunohistology, or by measuring sialyltransferase activity, preferably by ELISA. Sialylation is preferably α2-6-linked sialic acid; Sialyltransferase is preferably α2-6 sialyltransferase (see Examples).

In some embodiments, the treatment with the antibody is performed in combination with another cancer therapy such as chemotherapy, non-chemotherapy with specific inhibitors, or combination thereof. Chemotherapy is preferably with fludarabine, cyclophosphamide, bendamustine, chlorambucil, or combination thereof; for example fludarabine and cyclophosphamide; bendamustine or chlorambucil. Non-chemotherapy with specific inhibitors is preferably with the Bruton tyrosine kinase inhibitor Ibrutinib, the PI3-kinase delta inhibitor Idelalisib, and the BCL-2 inhibitor Venetoclax., or combination thereof.

In some embodiments, the patient as risk factors or comorbidities. For example, risk factors for CLL include unmutated heavy light immunoglobulin chain [IGVH], chromosome 17p deletion, or mutations of TP53 (tumor protein p53).

The method according to the invention is a predictive method which will allows deciding whether treatment with an antibody mediating complement activation is adapted to a patient. As such, the method according to the invention is preferably performed on a patient before applying the treatment with the antibody (i.e., on a tumor sample collected from the patient before starting the antibody treatment), more preferably on a patient who had not already been treated for said cancer.

The method according to the invention can be used for stratification of patients. As such, in another embodiment, said method is performed on a group of patients in order to classify each patient as responsive or non-responsive prior to administration of the antibody treatment.

The present invention also provides the use of antibody-induced CDC and/or sialylation as biomarkers for predicting the responsiveness of a cancer patient to a treatment with an antibody mediating complement-activation as defined above. The biomarker is preferably sialylation, more preferably α2-6-linked sialic acid.

### Treatment

The present invention also provides a method for the treatment of cancer in a patient in need thereof, comprising the following steps:
a. Performing the method for predicting the responsiveness to treatment according to the present invention on a tumor sample from the cancer patient; and
b. If the patient is predicted as responsive to the treatment, treating said patient with the antibody mediating complement-activation.

The antibody may be administered alone or in combination with another anticancer therapy including for example chemotherapy and/or specific inhibitors. The specific inhibitors include in particular the Bruton tyrosine kinase inhibitor Ibrutinib, the PI3-kinase delta inhibitor Idelalisib, and the BCL-2 inhibitor Venetoclax.

If the patient is predicted as non-responsive to the treatment, the method preferably comprises, administering another treatment to the patient. The other treatment may be a sialylation inhibitor, preferably α2-6-linked sialic acid inhibitor, in combination with the antibody mediating complement-activation wherein the inhibitor and the antibody may be administered simultaneously, separately or sequentially.

Preferably, said antibody is RTX for the treatment of CLL and said tumor sample comprises B lymphocytes.

The sialylation inhibitor, preferably α2-6-linked sialic acid inhibitor, may be an inhibitor of sialyltransferase, a group of enzymes that facilitate the transfer of sialic acid onto glycoconjugates in the Golgi lumen. The inhibitor may be an inhibitor of sialyltransferase expression or activity. Preferably, an inhibitor of α2-6 sialyltransferase. For example, Antagomir is an inhibitor of sialyl transferase activity. Examples of α2-6 sialyltransferase inhibitors are disclosed in WO 2017/035501.

Alternatively, the sialylation inhibitor is a sialidase, an enzyme responsible for the removal of α-glycosidically linked sialic acid residues from carbohydrate portions of glycoproteins. Sialidases are disclosed for example in Miyagi et al., Prog. Mol. Biol. Transl. Sci. 2018, 156, 121-150.

The sialylation inhibitor may also be an inhibitor of sialyltransferase expression, preferably a specific shRNA or siRNA.

The sialic acid inhibitor is preferably targeted to tumor cells using appropriate drug delivery vehicle that are well-known in the art.

The present invention also provides a product containing an antibody mediating CDC, preferably Rituximab, and a sialylation inhibitor, preferably α2-6-linked sialic acid inhibitor, as a combined preparation for simultaneous, separate or sequential use in cancer therapy, preferably CLL therapy. The sialylation inhibitor may be a sialyltransferase inhibitor, preferably α2-6 sialyltransferase inhibitor as defined above.

The present invention will now be illustrated by the following examples and figures.

### Figures:

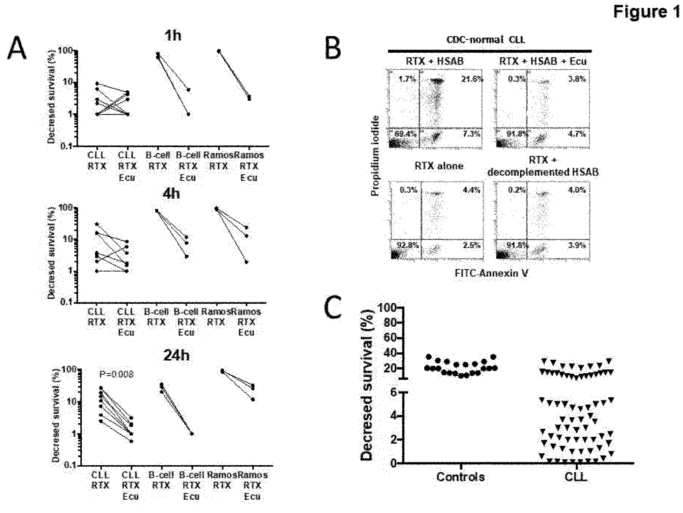

### Figure 1: Rituximab (RTX)-induces in vitro complement-dependent cytotoxicity (CDC) of B lymphocytes in normal controls and in a subset of CLL.

**A**. In order to improve the determination of CDC induction by RTX (10µg/ml) in CLL cells, CDC was initially established after 1h (top), 4h (middle), and 24h (bottom) incubation at 37°C in CLL cells (n=9), healthy control B cells (n=4), and the human B cell line Ramos (3 experiments) using normal human serum AB (HSAB, 20%) as a source of complement, and Eculizumab (Ecu, 10µg/ml) as a terminal complement inhibitor.
**B**. RTX capacity to induce CDC in a representative RTX-normal CLL patient was abrogated when adding Ecu, when complement present in HSAB was heat inactivated or when HSAB was omitted. C. B cells from 21 normal controls and 69 CLL patients were incubated 24h with RTX (10µg/ml) and HSAB at 20%. After staining with FITC-annexin V plus propidium iodide, cells were analyzed by FACS and the CDC decreased survival calculated (see material and methods).

### Figure 2: Response to therapy in CLL patients is associated with sensitivity to RTX-induced complement-dependent cytotoxicity (CDC)

**A**. TTR (time to relapse) of patients with B cells sensitive to RTX-induced CDC was compared to those resistant.
**B**. Overall survival (OS) of these two groups of patients was established and compared.

### Figure 3: Expression of CD20, ganglioside M1, sphingomyelin (SM), and complement inhibitors (CD55, CD59 and factor H) in healthy control B cells and CLL cells according to their complement-dependent cytoxicity (CDC) status to rituximab (RTX). 5x10⁵ B lymphocytes were incubated with

**A**. anti-CD20 antibody, cholera toxin B identifying GM1 and lysenin identifying sphingomyelin (SM).
**B**. Same experiments were conducted with saturated concentrations of anti-CD55, anti-CD59 and anti-factor H antibodies. For each staining, a representative example is shown on the left side. The shaded histogram corresponds to isotype control, the solid line to a representative B cell staining from the CDC-normal patient group, and the dotted line to a representative B cell staining from the CDC-resistant patient group. The number of samples tested is indicated in Table 2 and differences are indicated when p<0.05.

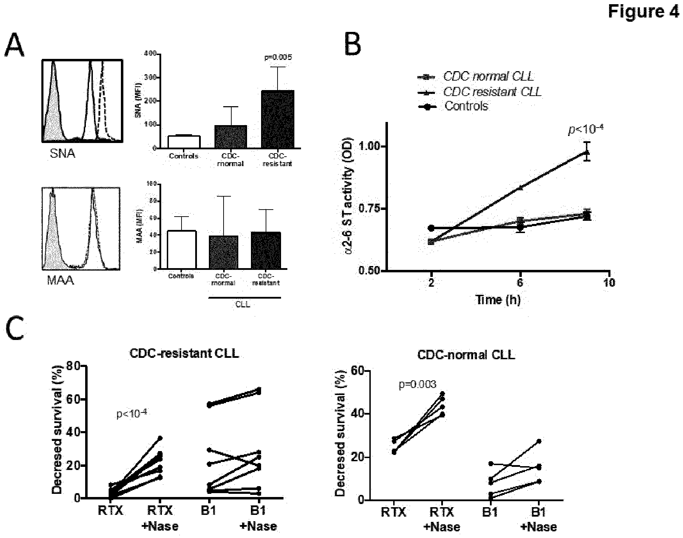

### Figure 4: Hypersialylation characterizes CLL cells resistant to rituximab (RTX) induced complement-dependent cytotoxicity (CDC).

**A**. FACS analysis of α2-6 linked sialic acid stained with the lectin *Sambucus nigra* (SNA, upper panel) and α2-3 linked sialic acid stained with *Maakia amureusis* (MAA, lower panel). For each staining, a representative example is shown on the left side. The shaded histogram corresponds to isotype control, the solid line to CLL cells sensitive to RTX CDC, and the dotted line to CDC-resistant cells.
**B**. B cell lysates from five CDC-resistant patients, five CDC-normal patients and to the four controls were incubated on ELISA plates coated with an acceptor of sialic acid for 2, 6 or 9 hours. After washes, the biotinylated lectin SNA specific for sialic acid linked to α2-6 sialic acid was added. The results were visualized after incubation of streptavidin-conjugated horseradish peroxydase and 1,2 ortho-phenylenediamine.
**C**. B lymphocytes from ten patients within the CDC-resistant group and five patients within the CDC-normal group were treated with or without 0.05U of neuraminidase from *Clostridium perfringens*, washed and incubated with or without 10µg/ml of RTX or 10µg/ml of B1 (tositumomab) for 24 hours at 37°C. CDC was quantified by FACS after staining with FITC-annexinV and propidium iodide. Differences are indicated when *p*<0.05.

### EXAMPLES

The following examples use the materials & methods described below:

### MATERIALS & METHODS

### Patients and normal controls

Sixty-nine untreated patients fulfilling CLL diagnostic criteria were enrolled in this retrospective study including 34 tested before the RTX chemotherapy initiation (0 to 7 months) [45]. Disease assessment included Binet stage determination, CD38 expression, lymphocyte counts, Lymphocyte doubling time (LDT), and cytogenetic analysis as previously described [46, 47]. Progression free survival (PFS) was defined as the time from disease discovery to disease progression. Disease progression was considered either as the shift from Binet stage A to Binet stage B/C or as a short LDT of less than 6 months. Treatment free survival (TFS) was defined as the interval between the date of disease discovery and the date of treatment initiation. Patients were segregated into cytogenetic risk groups according to the Döhner's classification [48]: patients with isolated del(13q) were in the low risk group, normal karyotype or trisomy 12 were in the intermediate risk group, and del(11q), del(17p) or complex karyotype were in the high risk group. RTX was introduced as first line treatment in combination with fludarabine and cyclophosphamide (RFC, n=28) or with bendamustine (RB, n=6). Response to therapy was assessed according to the 2008 IW-CLL guidelines [49] by determining the complete response (CR) at the end of RTX infusions, TTR was defined as the time from RTX initiation to the time of disease progression (≥5 ×10⁹ peripheral B cells/L), and the overall survival (OS) rate from the inclusion to death from any cause. Blood was also withdrawn from 21 healthy volunteers. Consent was obtained from all individuals and the protocol approved by the Ethical Board at the Brest University Medical School Hospital (OFICE, November 26^{th}, 2015; ClinicalTrials.gov: NCT03294980; CRB Brest collection 2008-214), in accordance with the Declaration of Helsinki).

### Specimen collections and complement analysis

Blood samples were collected in order to test the RTX capacity to induce CDC and for cellular analysis. Plasma was stored at -80°C until tested for C3c (normal range (NR): 0.81-1.57 g/L) and C4 (NR: 0.13-0.39 g/L) by turbidimetry (Spa-Plus®, The binding site group limited, Birmingham, UK).

### Cell preparation

Peripheral blood mononuclear cells were separated by density-gradient centrifugation on Ficoll-Hypaque, and B lymphocytes were isolated using a B-cell isolation kit according to the manufacturer's instructions (Stem Cell Technologies, Grenoble, France). The purity estimated by flow cytometry using FITC-conjugated anti-CD19 and PE-conjugated antiCD5 mAbs was over 95%.

### Complement-dependent cell cytotoxicity assay

Based on the preliminary step optimization **(****Figure 1**), RTX-induced CDC was evaluated *in vitro* as follows. Briefly, 3x10⁵ B lymphocytes were incubated into 24-well plates at 37°C in RPMI-1640 (Sigma-Aldrich, Saint-Quentin Fallavier, France) supplemented with 2mM L-glutamine, antibiotics, 10% fetal calf decomplemented serum (Gibco, Paisley, Scotland), and 20% of HSAB decomplemented or not 20 min at 56°C (Invitrogen, Cergy Pontoise, France). In all protocols 10µg/ml RTX-stimulation (Roche, Paris, France) (saturating Ab conditions [37]) with or without 10µg/ml Eculizumab (Soliris; Alexion Pharmaceuticals, Cheshire, CT), B lymphocytes were collected, washed and stained for 15 minutes with FITC-conjugated annexin-V (AV) and propidium iodide (PI) using the Beckman-Coulter apoptosis kit. The percentage of CDC decreased survival was calculated by using the formula: 100x [(% of AV-PI- cells without RTX in HSAB)-(% of AV-PI- cells with RTX in HSAB)]/[% of AV-PI- cells without RTX in HSAB]. In selected experiments, direct apoptosis and ADCC [25, 32] were monitored as previously described; and cells were additionally treated with 0.05U of neuraminidase (Sigma-Aldrich) for 30 minutes at 37°C, and two washes were then performed to avoid any side effects of neuraminidase on RTX or B1 (10µg/ml, generous gift of Cragg MS, Southampton) activity.

### Mutational status of IGHV

As previously described [68, 69], the *IGHV* gene mutation status was determined by sequencing after conducting a PCR multiplex amplification. Briefly, for multiplex PCR, 100ng of genomic DNA, 0.25µl of Ampli Taq Gold DNA Polymerase (Applied Biosystems, Foster City, CA), 10pmol of each primer, 0.2mM dNTP Mix, 1.5mM MgCl2, 1x PCR Buffer II, were adjusted to 50µl with DNase/RNase free ultrapure distilled water. Next, PCR products were visualized on 2% agarose gel, and purified with ExoSAP-IT PCR product cleanup kit (Affymetrix, High Wycombe, UK). Finally, amplicons were sequenced with a Big Dye Terminator v3.1 cycle sequencing kit (Applied Biosystems). Results were analyzed with the database IMGT/HighV-Quest (The international ImMunoGeneTics information system, Montpellier) and a homology sequence >98% defined an UM status.

### Polymorphism analysis in FcyR3A V158F

The BioSprint 15 DNA blood kit was used to extract genomic DNA from mononuclear cells according to the manufacturer's instructions (Qiagen, valencia, CA). Polymorphisms were determined as previously described [38, 31] using allele-specific polymerase chain reactions (PCRs) with one unmodified primer for FcγR3a-158V/F (ATATTTACAGAATGGCACAGG; SEQ ID NO: 1) and one locked nucleic acid modified primer for FcγR3a-158V: GAAGACACATTTTTACTCCCAA+C (SEQ ID NO: 2) *versus* for FcγR3a-158F CTCTGAAGACACA-TTTTTACTCCCAA+A (SEQ ID NO: 3).

### Flow cytometry

In this study, 5x10⁵ freshly isolated lymphocytes were incubated for 30 minutes at 4°C with saturating concentrations of anti-CD19 mAbs combined with phycoerythrin (PE)cyanin 7 (PCY7), and another mAb (anti-CD5, anti-CD20, anti-CD59 or anti-CD55) conjugated with fluorescein isothiocyanate (FITC). All mAb, as well as their isotype control mAb were from Beckman Coulter (Villepinte, France), biotinylated lectin SNA, specific to the α2-6 linked sialic acid, and biotinylated lectin MAA specific to the α2-3 linked sialic acid (Vector Burlingame, CA), lysenin recognizing SM (PeptaNova, Sandhausen, Germany) or FITC-conjugated cholera toxin subunit B recognizing GM1 (Sigma-Aldrich). Samples containing biotinylated-lectin were covered for an additional 30 minutes with FITC conjugated streptavidin (Jackson Immunoresearch Europe Ltd, Suffolk, UK). Cells stained with lysenin were incubated for 30 minutes with a polyclonal rabbit anti-lysenin Ab, and for another 30 minutes with FITC-conjugated anti-rabbit mAb (PeptaNova, Sandhausen, Germany). To study the binding of factor H on CLL cells, 3.10⁵ cells were incubated for one hour at 37°C with normal human serum diluted 1/4 in PBS followed by a 30-minute incubation with FITC-conjugated anti-factor H mAb (Abcam, Paris, France) at 4°C. The cells were then washed with phosphate buffer saline (PBS) and analyzed on FC500 flow cytometer (Beckman Coulter). The calculation of the MFI of all markers required a minimum of 5,000 events.
The number of CD20 molecules per cell was quantified by determining the amount of mAb binding to the cells at saturating concentrations, using the Quantum kit (Flow Cytometry Standards Corp).

### Assay for sialyltransferase activity

The activity of α2,6 and α2,3 sialyltransferases was evaluated by ELISA after B cell lysis as previously described [35] and the optical density (OD) was determined by a spectrometer ELISA at 492 nm.

### Statistical analysis

Continuous data are described as mean ± standard error of the mean (SEM). Following normality and equality of variance tests, nominal values were compared using the student's t test or alternatively by using a nonparametric test (Mann-Whitney rank sum test).

For categorical data, differences among groups were analyzed using the Fisher's exact test. The profile likelihood method using a Cox regression model of TTR was used in univariate analysis to determine the optimal threshold, this analysis was computed using the Survival and SurvMisc R packages [32]. LDT, TFS, PFS and TTR analyses were performed using Kaplan-Meier curves and prognosis differences between groups were assessed with a logrank test. P values under 0.05 were considered significant. Statistical analyses were performed using GraphPad Prism 7.0 (La Jolla, CA).

### Example 1: CDC of CLL cells induced by RTX is delayed and inhibited by a complement inhibitor (Eculizumab)

The RTX capacity to induce *in vitro* CDC has been addressed by several authors but with significant differences with regards to the source and number of B cells, the time and concentration of RTX, the amount of sera, and the method for CDC detection. Accordingly, the assay to test complement mediated killing of CLL cells sensitized by the anti-CD20 mAb RTX (10µg/ml) at 37°C was optimized in a preliminary step and, as shown in **Figure 1A**, CDC induced by RTX was effective after 1h when using the human B cell line Ramos (n=3) but such an effect was delayed to 24h when using CLL cells (n=8) and B cells from healthy controls (n=3). It was further established that the RTX capacity to lyse CLL cells disappeared when using Eculizumab as a terminal complement inhibitor, when complement present in HSAB (human serum AB) was heat-inactivated or when HSAB was omitted (**Figure 1** **A/B**). Next, in order to differentiate CDC-normal from CDC-resistant CLL cells, the 24h time point was selected and a cut-off value of 6% was established by considering the mean of decrease in survival (21.2±7.6%) minus 2 standard deviations of normal B cells from 21 healthy controls (**Figure 1C**). Therefore, 45 out of 69 (65.2%) CLL patients were considered resistant to complement *in vitro* and this is independent from the RTX capacity to induce direct apoptosis and ADCC (**Table 1**).

**Table 1: Complement dependent cytoxicity (CDC), direct apoptosis, antibody dependent cell cytotoxicity (ADCC) and associated factors characteristic for CDC-normal and CDC-resistant chronic lymphocytic leukemia (CLL) patients**

| | **Control B cells** | **CDC-normal CLL** | **CDC-resistant CLL** | **Normal *vs* Resistant** |
|---|---|---|---|---|
| Decreased survival (%, CDC) | 21.2±1.7 (21)* | 16.1±1.4 (24) | 2.0±0.2 (45) | *p*<10⁻³ |
| Decreased survival (%, apoptosis) | 4.4±2.8 (8) | 5.9±1.6 (9) | 4.3±1.3 (8) | NS |
| Decreased survival (%, ADCC) | | 13.6±1.6 (6) | 16.1±5.6 (6) | NS |
| CD20 (ABC units) | 317,000±56,800 (6) | 95,700±12,000 (9) | 75,000±5,800 (39) | NS |
| GM1 (MFI) | 2.5±0.25 (6) | 2.6±0.2 (20) | 2.3±0.3 (26) | NS |
| Sphingomyelin (MFI) | 33.4±3.2 (6) | 27.5±7.8 (8) | 31.1±5.0 (16) | NS |
| CD55 (MFI) | 11.6±0.9 (6) | 8.7±1.6 (6) | 10.7±2.1 (6) | NS |
| CD59 (MFI) | 5.5±0.7 (6) | 5.6±0.8 (6) | 6.8±1.0 (6) | NS |
| Factor H binding (%) | 20.4±4.8% (6) | 13.3±3.0% (6) | 18.3±4.2% (10) | NS |
| Sambucus nigra (MFI) | 52±3 (6) | 97±32 (6) | 243±35 (8) | *p*=0.01 |
| Maakia amurensis (MFI) | 44.8±8.5 (6) | 38.6±17.8 (7) | 43.4±5.1 (28) | NS |
| α2-6 ST activity (OD, 9h) | 0.721±0.011 (4) | 0.730±0.009 (5) | 0.981±0.017 (5) | *p*<10⁻⁴ |

| | | | | |
|---|---|---|---|---|
| Abbreviations: ND: not determined; ABC: antibody-binding capacities; MFI: mean fluorescence intensity; OD: optical density at 492nm, NS: not significant. *The number of samples tested is indicated in brackets. | | | | |

### Example 2: Time to relapse (TTR) is reduced in the in vitro CDC-resistant CLL patient subgroup

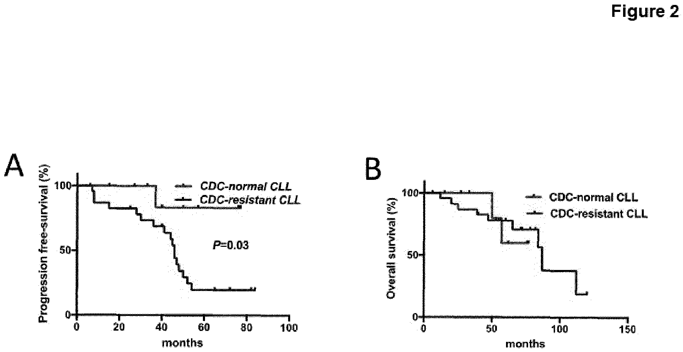

When the whole population was taken into consideration **(Table 2**), no difference was observed between the CDC-resistant and -normal groups with regards to demographic, clinical and biological characteristics of the patients (age, sex, Binet stage, progression free survival [PFS], treatment free survival [TFS], lymphocytosis, lymphocyte doubling time [LDT], cytogenetic characteristics, and CD38 expression). Further on, in patients tested before the initiation of RTX-chemotherapy for CDC (n=34), response to therapy was evaluated revealing in the CDC-resistant group a reduction when considering complete response (CR) (65.2% in the CDC-resistant patient group *versus* 100% in the CDC-normal patients group, p=0.03), and TTR (median TTR: 45 months in the CDC-resistant patient group *versus* >80 months in the CDC-normal patient group; Hazard Ratio 7.2, 95% CI 2.6-19.8, *p*=0.03) (**Figure 2** **and Table** 2). The Cox regression model was used to determine the optimal cutoff for the CDC assay that was 6.7% and close to 6% (mean control minus 2 standard deviation). In contrast, overall survival (OS) difference between the two groups was not significant due to the development of a secondary myelodysplastic syndrome leading to death in two patients from the CDC-normal patient group. Interestingly, our results further support an over-representation of CLL with unmutated IGHV in the *in vitro* CDC-resistant group (*p*=0.04), which is in agreement with Chai-Adisaksopha C and Brown JR report [28].

**Table 2: Characteristics of the patients according to rituximab capacity to induce complement-dependent cytotoxicity (CDC)**

| | **CDC-normal** (**n=24 including 11 treated**) | **CDC-resistant** (**n=45 including 23 treated**) | **Statistics** |
|---|---|---|---|
| **Demographic factors** | | | |
| Age, mean±SEM | 73±2 | 69±2 | NS |
| Sex Male:Female | 10:14 | 27:18 | NS |
| **Clinical data** | | | |
| Binet A/B/C | 7/13/4 | 13/23/9 | NS |
| PFS, mean (months)* | 53 | 72 | NS |
| TFS, mean (months)* | 96 | 108 | NS |
| **Tumoral cells** | | | |
| Lymphocytosis | 80±12 | 69±8 | NS |
| LDT, mean (months)* | 10 | 23 | NS |
| *CD38* >*30%, n (%)* | 6/22 (27.3%) | 10/43 (23.3%) | NS |
| **Cytogenetics, n (%)** | | | NS |
| *Low risk* | *9*/*21 (42.9%)* | *18*/*39 (46.2%)* | |
| *Intermediate risk* | 5/21 (23.8%) | 6/39 (15.4%) | |
| *High risk* | 7/21 (33.3%) | 15/39 (38.5%) | |
| **Therapeutic response** | | | |
| Complete response, n (%) | 11/11 (100%) | 15/23 (65.2%) | *p*=0.03 |
| Partial response, n (%) | 0/11 | 8/23 (34.8%) | |
| TTR, mean (months)* | >80 | 45 | *p*=0.03 |
| OS, mean (months)* | >80 | 87 | NS |
| **Cytogenetics, n (%)** | | | |
| *Low risk* | 5/11 (45%) | 5/20 (25%) | NS |
| *Intermediate risk* | 2/11 (18%) | 4/20 (20%) | |
| *High risk* | 4/11(37%) | 11/20 (55%) | |
| **Complement fractions at 1^{st} infusion** | | | |
| C3c low level | 2/10 | 4/19 | NS |
| C4 low level | 2/10 | 0/19 | NS |
| **IGHV UM/M** | 0/5 | 6/8 | *p*=0.04 |
| **FcγR3A V158F**, **n** (%) | | | |
| FF | 5/10(50%) | 11/20 (55%) | NS |
| VF | 4/10 (40%) | 8/20 (40%) | |
| VV | 1/10 (10%) | 1/20 (5%) | |
| **Treatment** | | | |
| RFC:RB | 8:3 | 20:3 | NS |

| | | | |
|---|---|---|---|
| Abbreviations: PFS: Progression free survival; TFS: Treatment free survival; LDT: Lymphocyte doubling time; n: number of patients; NS: not significant; RFC: rituximab fludarabine and cyclophosphamide; RB: rituximab bendamustine; TTR: time to relapse; OS: overall survival. *Kaplan-Meyer survival analysis. | | | |

### Example 3: Rituximab incapacity to induce CDC is not related to complement inhibitor levels, CD20 expression, FcγR3A V158F polymorphism or lipid membrane disorganization

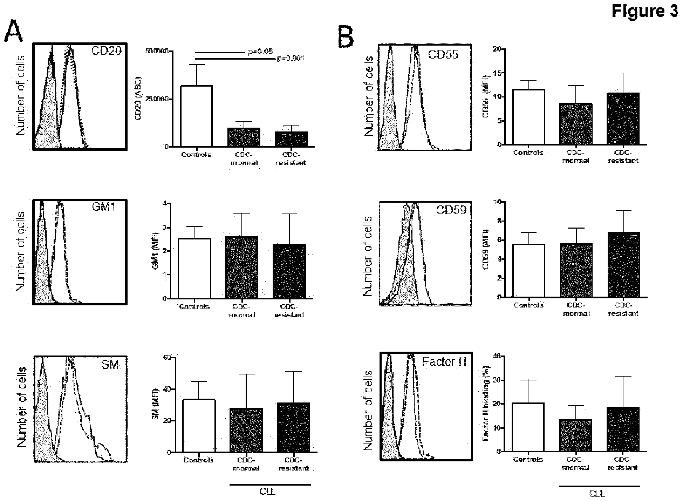

CD20 (target antigen), GM1 and sphingomyelin (lipid raft markers) *in vitro* expression were compared between the two patient groups as the ability of CD20 to translocate into lipid rafts is suspected to be important for complement activation by antiCD20 mAb [29], and that lipid raft integrity may be compromised in CLL cells [25,26,32] (**Figure 3A** **and Table 1**). No differences were observed when comparing in both groups (i) the numbers of CD20 molecules between groups; (ii) the mean fluorescence intensity (MFI) of the FITC-conjugated cholera toxin subunit B recognizing GM1; and (iii) the MFI of lysenin-bound-sphingomyelin. As the inventors have previously observed that sphingomyelin overexpression in CLL cells induced by rifampicin affects the type II anti-CD20 mAb (B1) capacity to kill CLL cells [30], the experiment was repeated with RTX instead of B1 (tositumomab), revealing that sphingomyelin overexpression had no effect on the RTX capacity to induce CDC. Regarding complement regulators, the CLL cell surface expression of the complement inhibitors CD55 and CD59 was further explored [33], and CLL capacity to recruit the fluid-phase factor H, another RTX-mediated CDC inhibitor [34] (**Figure 3B** **and Table 1**). The MFI of CD55, CD59 and the percentage for factor H binding were assessed revealing similar values between the *in vitro* CDC-normal patient group and the CDC resistant patient group. In order to complete such analysis, the complement deficiency at RTX-chemotherapy initiation was based on observing C3c and C4, as well as the cytogenetic status and FcγR3A V158F polymorphism known to be critical for proper CDC and ADCC, respectively (**Table 2**). No difference was reported and all these factors were not considered further.

### Example 4: CLL lymphocytes CDC-resistant to rituximab express higher levels of terminal α 2-6 linked sialic acids and their removal by neuraminidase increases activity of rituximab induced CDC

The presence of α2-3 and α2-6-linked sialic acid was tested on CLL cells and controls using specific lectins. For this purpose, two lectins from *Sambucus nigra* (SNA) and from *Maakia amurensis* (MAA) were selected, which bind to sialic acid attached to terminal galactose in α2-6 and in α2-3, respectively (**Figure 4A**). The CLL cell membranes of the CDC-resistant patient group expressed more α2-6 sialylated proteins (MFI of 243±35) than those of the CDC-normal patient group (MFI of 97±32, *p*=0.01), and controls (MFI of 52±3). No differential expression of the lectin MAA was seen in the three different groups, suggesting similar levels of α2-3 sialylated proteins.

To test the hypothesis that α2-6 hypersialylation in the CDC-resistant patient group resulted from increased activity of a2-6 sialyl transferase (ST), the inventors used a previously developed custom-made ELISA in order to measure the α2-6 ST activity in CLL cells [35].
As shown in **Figure 4B****,** the α2-6 ST activity was found to be significantly upregulated in the CDC-resistant CLL cells treated (0.981±0.017) compared to the CDC-normal CLL cells (0.730±0.009, *p*<10⁻⁴) and to the B cell controls (0.721± 0.011, *p*<10⁻⁴). In the MAA binding assay, the α2-3 ST activity was similar in the three groups (data not shown). Finally, and to strengthen the effect of sialic acid on RTX-mediated CDC, the inventors further selected 10 CLL patients from the CDC-resistant group and 5 patients from the CDC normal group and cells were treated with neuraminidase to remove sialic acids from cell surface. In the CDC-resistant group, following neuraminidase treatment, RTX capacity to induce CDC was restored (*p*<10⁻⁴) (**Figure 4C**). Interestingly, the neuraminidase capacity to increase CDC activity was not restricted to the CDC-resistant subgroup as the same observation was made in the CDC-normal patient group (*p*=0.003). As a control and to test RTX specificity, when using B1 (tositumomab) instead of RTX, B1 capacity to directly kill CLL cells was similar following or not neuraminidase treatment. Furthermore, since one may argue that desialylation can lead to membrane modifications and possibly to better accessibility of RTX to CD20 [36], the inventors looked at the binding of RTX on the cell surface after desialylation. MFI of FITC-conjugated RTX binding to CD20 was similar with or without treatment with neuraminidase (data not shown). Altogether, these results shows that treating CLL cells with neuraminidase can restore *in vitro* CDC induced by RTX, and differences between the two groups are related to quantitative and/or qualitative differences in SA.

### CONCLUSIONS

The RTX capacity to induce CDC was established in 69 untreated CLL patients, this cohort including 34 patients tested before the initiation of RTX-chemotherapy. *In vitro* CDC-resistance to RTX predicts lower response rates to RTX chemotherapy and shorter treatment free survival. Furthermore, the predictive value of CDC-resistance was independent from the clinical, cytogenetic (chromosome deletion 13q, 17p, 11q and trisomy 12) and FcγR3A V158F polymorphism status. In contrast, CLL cell resistance to CDC predominates in IGHV unmutated patients and was surprisingly related to an important α2-6 sialyl transferase activity, which in turn increases cell surface α2-6 hypersialylation. Suspected factors associated with resistance to CDC (CD20, CD55, CD59, factor H, GM1, and sphingomyelin) were not differentially expressed or recruited between the two CLL groups. Altogether, the results provide evidence that testing RTX capacity to induce CDC *in vitro* represents an independent predictive factor of therapeutic effects of RTX, and that α2-6 hypersialylation in CLL cells controls RTX response through the control of the complement pathway.

The inventors measured *in vitro* the capacity of RTX to induce CDC. Tumor cell lines sensitive to RTX, specific inhibitor of complement activity and different times of RTX incubation were used. Based on the *in vitro* capacity of RTX to induce the complement pathway, two groups of patients were established. The first group, presenting a complete response (CR) (100%) and a delayed median time to relapse (TTR) over 80 months, was sensitive to CDC *in vitro,* while the second group, with a reduced CR rate (65.2%) and a median TTR at 45 months, was resistant to CDC *in vitro.* Of note, TTR in the CDC-resistant group was similar to the TTR reported in 409 treatment-naive CLL patients receiving fludarabine and cyclophosphamide [39]. Moreover, the higher rate of CR at the end of treatment in the CDC-normal patient group (100% versus 65.2%) further supports within the CDC-resistant patient group an incomplete capacity of RTX to activate the complement pathway.

After determining that a longer culture time (24h) was required to allow CDC determination in CLL cells and having confirmed the absence of CDC response when using the anti-C5 mAb Eculizumab acting downstream C3 activation or when the human serum AB (HSAB) was omitted or decomplemented by heat, important complement resistance mechanisms were suspected in CLL cells. After observing that CDC was restored in CLL cells after treatment with neuraminidase, it was alluring to incriminate sialylation as involved mechanism. Sialic acid is used in cancers, such as breast and ovarian carcinoma, as a protection against complement activity [29], and removal of sialic acid from the cell surface by sialidase increases sensitivity to CDC [40-42]. Applied to CLL patients, the inventors further established that the α2-6 sialylation level influences resistance to the RTX-induced CDC patient group, and predicts the in vivo clinical RTX-chemotherapy outcome. More specifically, the inventors established that CDC is influenced by the level of cell surface α2-6-linked sialic acid and that a stronger activity of α2-6 sialiltransferase characterizes the CDC-resistant patient group.

Also, the inventors established that the resistant capacity to CDC is not associated with distinct individual genetic groups and clinical outcome.

Accordingly, the results support an abnormal quantity of sialic acid available on cancer cells, preferably CLL cells to control complement-activation mediated by RTX.

### REFERENCES

1. Robak T, Lech-Maranda E, Robak P. Rituximab plus fludarabine and cyclophosphamide or other agents in chronic lymphocytic leukemia. Expert Rev Anticancer Ther. 2010; 10:1529-1543.
2. Huhn D, von Schilling C, Wilhelm M, Ho AD, Hallek M, Kuse R, Knauf W, Riedel U, Hinke A, Srock S, Serke S, Peschel C, Emmerich B, et al. Rituximab therapy of patients with B-cell chronic lymphocytic leukemia. Blood. 2001; 98:1326-1331.
3. Cramer P, Langerbeins P, Eichhorst B, Hallek M. Advances in first-line treatment of chronic lymphocytic leukemia: current recommendations on management and first-line treatment by the German CLL Study Group (GCLLSG). Eur J Haematol. 2015; 96:9-18.
4. Bagacean C, Zdrenghea M, Tempescul A, Cristea V, Renaudineau Y. Anti-CD20 monoclonal antibodies in chronic lymphocytic leukemia: from uncertainties to promises. Immunotherapy. 2016; 8:569-581.
5. Clynes RA, Towers TL, Presta LG, Ravetch JV. Inhibitory Fc receptors modulate in vivo cytotoxicity against tumor targets. Nat Med. 2000; 6:443-446.
6. Veeramani S, Wang SY, Dahle C, Blackwell S, Jacobus L, Knutson T, Button A, Link BK, Weiner GJ. Rituximab infusion induces NK activation in lymphoma patients with the high-affinity CD16 polymorphism. Blood. 2011; 118:3347-3349.
7. Di Gaetano N, Cittera E, Nota R, Vecchi A, Grieco V, Scanziani E, Botto M, Introna M, Golay J. Complement activation determines the therapeutic activity of rituximab in vivo. J Immunol. 2003; 171:1581-1587.
8. Zent CS, Elliott MR. Maxed out macs: physiologic cell clearance as a function of macrophage phagocytic capacity. FEBS J. 2017; 284:1021-1039.
9. Grandjean CL, Montalvao F, Celli S, Michonneau D, Breart B, Garcia Z, Perro M, Freytag O, Gerdes CA, Bousso P. Intravital imaging reveals improved Kupffer cell-mediated phagocytosis as a mode of action of glycoengineered anti-CD20 antibodies. Sci Rep. 2016; 6:34382.
10. Gul N, Babes L, Siegmund K, Korthouwer R, Bogels M, Braster R, Vidarsson G, ten Hagen TL, Kubes P, van Egmond M. Macrophages eliminate circulating tumor cells after monoclonal antibody therapy. J Clin Invest. 2014; 124:812-823.
11. Montalvao F, Garcia Z, Celli S, Breart B, Deguine J, Van Rooijen N, Bousso P. The mechanism of anti-CD20-mediated B cell depletion revealed by intravital imaging. J Clin Invest. 2013; 123:5098-5103.
12. Cartron G, Dacheux L, Salles G, Solal-Celigny P, Bardos P, Colombat P, Watier H. Therapeutic activity of humanized anti-CD20 monoclonal antibody and polymorphism in IgG Fc receptor FcgammaRIIIa gene. Blood. 2002; 99:754-758.
13. Anolik JH, Campbell D, Felgar RE, Young F, Sanz I, Rosenblatt J, Looney RJ. The relationship of FcgammaRIIIa genotype to degree of B cell depletion by rituximab in the treatment of systemic lupus erythematosus. Arthritis Rheum. 2003; 48:455-459.
14. Kim DH, Jung HD, Kim JG, Lee JJ, Yang DH, Park YH, Do YR, Shin HJ, Kim MK, Hyun MS, Sohn SK. FCGR3A gene polymorphisms may correlate with response to frontline R-CHOP therapy for diffuse large B-cell lymphoma. Blood. 2006; 108:2720-2725.
15. Hatjiharissi E, Xu L, Santos DD, Hunter ZR, Ciccarelli BT, Verselis S, Modica M, Cao Y, Manning RJ, Leleu X, Dimmock EA, Kortsaris A, Mitsiades C, et al. Increased natural killer cell expression of CD16, augmented binding and ADCC activity to rituximab among individuals expressing the Fc{gamma}RIIIa-158 V/V and V/F polymorphism. Blood. 2007; 110:2561-2564.
16. Treon SP, Hansen M, Branagan AR, Verselis S, Emmanouilides C, Kimby E, Frankel SR, Touroutoglou N, Turnbull B, Anderson KC, Maloney DG, Fox EA. Polymorphisms in FcgammaRIIIA (CD16) receptor expression are associated with clinical response to rituximab in Waldenstrom's macroglobulinemia. J Clin Oncol. 2005; 23:474-481.
17. Dall'Ozzo S, Tartas S, Paintaud G, Cartron G, Colombat P, Bardos P, Watier H, Thibault G. Rituximab-dependent cytotoxicity by natural killer cells: influence of FCGR3A polymorphism on the concentration-effect relationship. Cancer Res. 2004; 64:4664-4669.
18. Bannerji R, Kitada S, Flinn IW, Pearson M, Young D, Reed JC, Byrd JC. Apoptotic-regulatory and complement-protecting protein expression in chronic lymphocytic leukemia: relationship to in vivo rituximab resistance. J Clin Oncol. 2003; 21:1466-1471.
19. Terui Y, Sakurai T, Mishima Y, Mishima Y, Sugimura N, Sasaoka C, Kojima K, Yokoyama M, Mizunuma N, Takahashi S, Ito Y, Hatake K. Blockade of bulky lymphoma-associated CD55 expression by RNA interference overcomes resistance to complement-dependent cytotoxicity with rituximab. Cancer Sci. 2006; 97:72-79.
20. Harjunpaa A, Junnikkala S, Meri S. Rituximab (anti-CD20) therapy of B-cell lymphomas: direct complement killing is superior to cellular effector mechanisms. Scand J Immunol. 2000; 51:634-641.
21. Seret G, Hanrotel C, Bendaoud B, Le Meur Y, Renaudineau Y. Homozygous FCGR3A-158F mutation is associated with delayed B-cell depletion following rituximab but with preserved efficacy in a patient with refractory lupus nephritis. Clin Kidney J. 2013; 6:74-76.
22. Golay J, Zaffaroni L, Vaccari T, Lazzari M, Borleri GM, Bernasconi S, Tedesco F, Rambaldi A, Introna M. Biologic response of B lymphoma cells to anti-CD20 monoclonal antibody rituximab in vitro: CD55 and CD59 regulate complement-mediated cell lysis. Blood. 2000; 95:3900-3908.
23. Mankai A, Bordron A, Renaudineau Y, Martins-Carvalho C, Takahashi S, Ghedira I, Berthou C, Youinou P. Purine-rich box-1-mediated reduced expression of CD20 alters rituximab-induced lysis of chronic lymphocytic leukemia B cells. Cancer Res. 2008; 68:7512-7519.
24. Meyer zum Buschenfelde C, Feuerstacke Y, Gotze KS, Scholze K, Peschel C. GM1 expression of non-Hodgkin's lymphoma determines susceptibility to rituximab treatment. Cancer Res. 2008; 68:5414-5422.
25. Keppler OT, Moldenhauer G, Oppenlander M, Schwartz-Albiez R, Berger EG, Funderud S, Pawlita M. Human Golgi beta-galactoside alpha-2,6-sialyltransferase generates a group of sialylated B lymphocyte differentiation antigens. Eur J Immunol. 1992; 22:2777-2781.
26. Meri S, Pangburn MK. Discrimination between activators and nonactivators of the alternative pathway of complement: regulation via a sialic acid/polyanion binding site on factor H. Proc Natl Acad Sci USA. 1990; 87:3982-3986.
27. Donin N, Jurianz K, Ziporen L, Schultz S, Kirschfink M, Fishelson Z. Complement resistance of human carcinoma cells depends on membrane regulatory proteins, protein kinases and sialic acid. Clin Exp Immunol. 2003; 131:254-263.
28. Chai-Adisaksopha C, Brown JR. FCR achieves long-term durable remissions in patients with IGHV-mutated CLL. Blood. 2017; 130:2278-2282.
29. Cragg MS, Morgan SM, Chan HT, Morgan BP, Filatov AV, Johnson PW, French RR, Glennie MJ. Complement-mediated lysis by anti-CD20 mAb correlates with segregation into lipid rafts. Blood. 2003; 101:1045-1052.
30. Hammadi M, Youinou P, Tempescul A, Tobon G, Berthou C, Bordron A, Pers JO. Membrane microdomain sphingolipids are required for anti-CD20-induced death of chronic lymphocytic leukemia B cells. Haematologica. 2012; 97:288-296.
31. Cornec D, Tempescul A, Querellou S, Hutin P, Pers JO, Jamin C, Bendaoud B, Berthou C, Renaudineau Y, Youinou P. Identification of patients with indolent B cell lymphoma sensitive to rituximab monotherapy. Ann Hematol. 2012; 91:715-721.
32. Borgan Ø. Modeling Survival Data: Extending the Cox Model. Terry M. Therneau and Patricia M. Grambsch, Springer-Verlag, New York, 2000. ISBN 0-387-98784-3. Stat Med. 2001; 20:2053-2054.
33. Takei K, Yamazaki T, Sawada U, Ishizuka H, Aizawa S. Analysis of changes in CD20, CD55, and CD59 expression on established rituximab-resistant B-lymphoma cell lines. Leuk Res. 2006; 30:625-631.
34. Horl S, Banki Z, Huber G, Ejaz A, Windisch D, Muellauer B, Willenbacher E, Steurer M, Stoiber H. Reduction of complement factor H binding to CLL cells improves the induction of rituximab-mediated complement-dependent cytotoxicity. Leukemia. 2013; 27:2200-2208.
35. Basset C, Durand V, Mimassi N, Pennec YL, Youinou P, Dueymes M. Enhanced sialyltransferase activity in B lymphocytes from patients with primary Sjogren's syndrome. Scand J Immunol. 2000; 51:307-311.
36. Dai J, Allard WJ, Davis G, Yeung KK. Effect of desialylation on binding, affinity, and specificity of 56 monoclonal antibodies against MUC1 mucin. Tumour Biol. 1998; 19 Suppl 1:100-110.
37. Bezombes C, Grazide S, Garret C, Fabre C, Quillet-Mary A, Muller S, Jaffrezou JP, Laurent G. Rituximab antiproliferative effect in B-lymphoma cells is associated with acid-sphingomyelinase activation in raft microdomains. Blood. 2004; 104:1166-1173.
38. Tempescul A, Bagacean C, Riou C, Bendaoud B, Hillion S, Debant M, Buors C, Berthou C, Renaudineau Y. Ofatumumab capacity to deplete B cells from chronic lymphocytic leukaemia is affected by C4 complement exhaustion. Eur J Haematol. 2016; 96:229-235.
39. Hallek M, Fischer K, Fingerle-Rowson G, Fink AM, Busch R, Mayer J, Hensel M, Hopfinger G, Hess G, von Grunhagen U, Bergmann M, Catalano J, Zinzani PL, et al. Addition of rituximab to fludarabine and cyclophosphamide in patients with chronic lymphocytic leukaemia: a randomised, open-label, phase 3 trial. Lancet. 2010; 376:1164-1174.
40. Lauf PK. Immunological and physiological characteristics of the rapid immune hemolysis of neuraminidase-treated sheep red cells produced by fresh guinea pig serum. J Exp Med. 1975; 142:974-988.
41. Jacobsen F. Increase of the in vitro complement-dependent cytotoxicity against autologous invasive human bladder tumor cells by neuraminidase treatment. Acta Pathol Microbiol Immunol Scand C. 1982; 90:187-192.
42. Turianskyj FH, Gyenes L. The effect of neuraminidase on the sensitivity of tumor cells toward lysis by antibody and complement or by sensitized lymphocytes. Transplantation. 1976; 22:24-30.
43. Weng WK, Levy R. Expression of complement inhibitors CD46, CD55, and CD59 on tumor cells does not predict clinical outcome after rituximab treatment in follicular non-Hodgkin lymphoma. Blood. 2001; 98:1352-1357.
44. Horl S, Banki Z, Huber G, Ejaz A, Mullauer B, Willenbacher E, Steurer M, Stoiber H. Complement factor H-derived short consensus repeat 18-20 enhanced complement-dependent cytotoxicity of ofatumumab on chronic lymphocytic leukemia cells. Haematologica. 2013; 98:1939-1947.
45. Matutes E, Owusu-Ankomah K, Morilla R, Garcia Marco J, Houlihan A, Que TH, Catovsky D. The immunological profile of B-cell disorders and proposal of a scoring system for the diagnosis of CLL. Leukemia. 1994; 8:1640-1645.
46. Bagacean C, Le Dantec C, Berthou C, Tempescul A, Saad H, Bordron A, Zdrenghea M, Cristea V, Douet-Guilbert N, Renaudineau Y. Combining cytogenetic and epigenetic approaches in chronic lymphocytic leukemia improves prognosis prediction for patients with isolated 13q deletion. Clin Epigenetics. 2017; 9:122.
47. Bagacean C, Tempescul A, Le Dantec C, Bordron A, Mohr A, Saad H, Olivier V, Zdrenghea M, Cristea V, Cartron PF, Douet-Guilbert N, Berthou C, Renaudineau Y. Alterations in DNA methylation/demethylation intermediates predict clinical outcome in chronic lymphocytic leukemia. Oncotarget. 2017; 8:65699-65716.
48. Dohner H, Stilgenbauer S, Benner A, Leupolt E, Krober A, Bullinger L, Dohner K, Bentz M, Lichter P. Genomic aberrations and survival in chronic lymphocytic leukemia. N Engl J Med. 2000; 343:1910-1916.
49. Hallek M, Cheson BD, Catovsky D, Caligaris-Cappio F, Dighiero G, Dohner H, Hillmen P, Keating MJ, Montserrat E, Rai KR, Kipps TJ, International Workshop on Chronic Lymphocytic L. Guidelines for the diagnosis and treatment of chronic lymphocytic leukemia: a report from the International Workshop on Chronic Lymphocytic Leukemia updating the National Cancer Institute-Working Group 1996 guidelines. Blood. 2008; 111:5446-5456.

## Claims

1. A method for predicting the responsiveness of a cancer patient to a treatment with an antibody or derivative thereof mediating complement-activation, comprising:
measuring the level of CDC induced by the antibody or its derivative or the level of sialylation in a tumor sample from the patient;
wherein if said level of CDC is decreased compared to a control, then the cancer patient is likely to be non-responsive to said treatment, if not then the cancer patient is likely to be responsive to said treatment; and/or
wherein if said level of sialylation is increased compared to a control, then the cancer patient is likely to be non-responsive to said treatment, if not then the cancer patient is likely be responsive to said treatment.

2. The method according to claim 1, wherein said cancer is selected from the group consisting of: leukemia, preferably chronic lymphocytic leukemia; lymphoma, preferably non-Hodgkin's lymphomas; myeloma, breast cancer, ovarian cancer, colorectal cancer, prostate cancer, renal carcinoma and melanoma.

3. The method according to any of claims 1 or 2, wherein said antibody is selected from IgG1 and IgG3 monoclonal antibody; preferably Type I anti-CD20, anti-CCR7, anti-CD52 anti-CD5, anti-CD38, anti-CD22, and anti-EGFR monoclonal antibodies.

4. The method according to claim 3, wherein said antibody is Rituximab.

5. The method according to any one of the preceding claims, wherein said antibody is Rituximab for the treatment of chronic lymphocytic leukemia and said tumor sample comprises B lymphocytes.

6. The method according to any one of the preceding claims, wherein said tumor sample has been collected prior to said treatment with the antibody.

7. The method according to any one of the preceding claims, wherein said sample is a whole blood sample, biopsy, or a sample of purified cancer cells.

8. The method according to any one of the preceding claims, which comprises measuring cell-surface sialylation, preferably by flow cytometry or immunohistology, or measuring sialyltransferase activity, preferably by ELISA.

9. The method according to claim 8, wherein said sialylation is α2-6-linked sialic acid or said sialyltransferase is α2-6-sialyltransferase.

10. The method according to any one of the preceding claims, wherein the control is:
a) a sample of normal cells from healthy controls or normal cell lines, or a sample of cells from a responsive patient, tested in the same conditions as the tested cancer patient, preferably said sample is from the same tissue as the tumor; or
b) the control for CDC level is a cut-off value established as mean value minus 2 standard deviations of a group of controls as in a) and the control for sialylation level is a cut-off value established as mean value plus 2 standard deviations of a group of controls as in a).

11. The method according to any one of the preceding claims, wherein said antibody treatment is performed in combination with chemotherapy, preferably with fludarabine and cyclophosphamide, with non-chemotherapy specific inhibitors, or combination thereof.

12. The method according to any one of the preceding claims, wherein said method is performed on a group of patients in order to classify each patient as responsive or non-responsive prior to the treatment.

13. Use of α2-6-linked sialic acid as a biomarker for predicting the responsiveness of a cancer patient to a treatment with an antibody mediating complement-activation.

14. A product comprising an antibody mediating complement-activation, preferably Rituximab, and a sialylation inhibitor, preferably a α2-6-linked sialic acid inhibitor, as a combined preparation for simultaneous, separate or sequential use in cancer therapy, preferably chronic lymphocytic leukemia therapy.
